# EUROPÄISCHE PATENTANMELDUNG

(11) **EP 2 308 559 A2**
(43) Veröffentlichungstag der Anmeldung: **13.04.2011**
(21) Anmeldenummer: 10013500.3
(22) Anmeldetag: 11.10.2010
(51) Int. Cl.: A61N 2/02

(54) **Sitz- oder Liegevorrichtung mit integrierter Schaltung zur Magnetfeldtherapie**

(30) Priorität: 09.10.2009 DE 202009013768 U
(71) Anmelder: Pichler, Christian, 5020 Salzburg (AT)
(72) Erfinder: Pichler, Christian, 5020 Salzburg (AT)
(74) Vertreter: Peters, Hajo

(57) **Zusammenfassung**

Eine Sitz- oder Liegevorrichtung zur Erzeugung eines Magnetfeldes zur Magnetfeldtherapie umfasst:
a) mindestens einen Aufnahmebereich;
b) eine Einrichtung, umfassend eine elektrische Leiterbahn, die zur bipolaren Stromerregung zur Erzeugung periodischer modulierter Magnetfelder im Aufnahmebereich eingerichtet ist;
c) eine aktive Brückenschaltung zur Steuerung der bipolaren Stromerregung zur Erzeugung periodischer modulierter Magnetfelder;
d) eine programmierbare Steuerung, die die Steuerung der Erzeugung periodischer modulierter Magnetfelder ermöglicht:
e) optional einen Sensor, der bevorzugt mit der Einrichtung nach b), der aktiven Brückenschaltung nach c) und/oder der Steuerung nach d) verbunden ist;
f) optional eine Energiequelle, die bevorzugt mit der der Einrichtung nach b), der aktiven Brückenschaltung nach c), der Steuerung nach d) und/oder dem Sensor nach e) verbunden ist.

## Beschreibung

Die vorliegende Erfindung betrifft eine Sitz- oder Liegevorrichtung mit integrierter Schaltung zur Erzeugung eines Magnetfeldes zur Magnetfeldtherapie. Die erfindungsgemäße Sitzoder Liegevorrichtung umfasst insbesondere eine aktive Brückenschaltung für bipolare Stromerregung, die in der Lage ist, periodische modulierte Magnetfelder zu erzeugen und einen Sensor, der eine automatisierte Durchführung der Magnetfeldtherapie erlaubt. Die erfindungsgemäße Sitz- oder Liegevorrichtung kann weiterhin als bewegliche Auflage für Sitz- oder Liegevorrichtungen gestaltet werden.

Die Mobilität der heutigen Gesellschaft erfordert heutzutage die Oberwindung immer längerer Distanzen per Auto, Bahn oder Flugzeug und damit auch immer längere Sitz- und Stressperioden für den menschlichen Körper. Besonders bei Interkontinentalflügen besteht die Gefahr von Thrombosen und Verspannungen, v.a. durch die unkomfortable und zumeist enge Sitzposition für Passagiere bei solchen Reisen. Dazu kommen in vielen Fällen der Wechsel von Zeitzonen und der durch solche Stressperioden verursachte und/oder verstärkte Jetlag. Ähnlich wie bei Flugreisen machen sich auch bei Bahnreisen und insbesondere Autoreisen solche durch Stressperioden induzierten Effekte bemerkbar. Gerade das Auto stellt für berufstätige Menschen ein Ort dar, an dem diese einen erheblichen Teil ihrer Zeit verbringen. Für den Fahrer oder Piloten ist es dabei vor allem erforderlich, ihre Konzentration und Aufmerksamkeit aufrechtzuerhalten und der körperlichen Ermüdung und Durchblutungsstörungen vorzubeugen. Für Passagiere in Flugzeug, Auto oder Bahn gilt dies in ähnlichem Maße, wobei gerade Passagiere die Zeit der Reise für die Zwecke der Erholung, zur Regeration und zum Stressabbau verwenden können. Auch bei Rollstuhlfahrer können vergleichbare Problem auftreten.

Im Stand der Technik werden zur Regeration und zum Stressabbau verschiedene Lösungen vorgeschlagen. So werden bspw. in modernen Sitzen für Kraftfahrzeuge teilweise aktive Massagesysteme eingebaut, um Teilbereiche des Körpers, beispielsweise den Rücken, in leichte Bewegung zu versetzen. Durch die Bewegung soll die Muskulatur angeregt und damit ein vorzeitiges Ermüden der Muskulatur verhindert werden, um gleichzeitig eventuell vorhandene Schmerzgefühle zu reduzieren.

Solche Massagesysteme haben jedoch häufig den Nachteil, dass sie bewegte Komponenten aufweisen, was den Einbau aufwändig und kostenintensiv macht und eine regelmäßige Wartung erfordert. Außerdem besitzen sie aufgrund der integrierten Antriebe üblicherweise ein hohes Gewicht und erfordern eine aufwändige Steuerung. Durch eine solche Vorrichtung wird ferner lediglich die Rückenmuskulatur aufgelockert und dadurch vor frühzeitiger Ermüdung geschützt. Andere Bereiche werden davon aber nicht erfasst. Es ist jedoch von erheblichem Interesse, dass das Wohlfühlempfinden der auf dem Sitz sitzenden Person allgemein gesteigert wird und so der gesamte Organismus vor vorzeitiger Ermüdung bewahrt wird.

Um dieses Problem zu lösen werden im Stand der Technik alternativ oder zusätzlich Magneten oder Magnetfelder zur Magnetfeldtherapie eingesetzt. Dabei hat sich die Magnetfeldtherapie in den letzten Jahren als innovative therapeutische Behandlung herausgestellt, die in vielen Fällen zu einer Verbesserung des Allgemeinzustandes der Patienten und in spezifischen Fällen zu unerwarteten Heilungserfolgen führte.

So wird bspw. der Stoffwechsel von Lebewesen, wie z.B. Säugetieren einschließlich des Menschen, auch über elektromagnetische Vorgänge gesteuert. Beispielsweise werden über elektrische Potenziale an der Zellmembran Ionen auch gegen Konzentrationsgefälle "gepumpt" (aktiver Stoffwechsel). Der Wasser- und Elektrolythaushalt des Körpers beruht zum Teil auf Phänomenen, die in Zusammenhang mit elektromagnetischer Wechselwirkung stehen. Die Kommunikation mit der Außenwelt, die Informationsübermittlung, die Tätigkeit und Koordination der inneren Organe sowie Muskelkontraktionen, einschließlich denen des Herzmuskels (EKG), erfolgen durch elektrische Erregung (NS). Ebenso beruhen die höhere Nerventätigkeit und die Funktionsfähigkeit des Gehirns auf Bioelektrizität (EEG). Magnetfelder als besondere elektromagnetische Wechselwirkungen können in einem Objekt, beispielsweise einem menschlichen Körper, Veränderungen verursachen, welche jenen der bei Bewegungsaktivitäten entstehenden Veränderungen gleichen und somit normale biologische Abläufe unterstützen können. Eine biologische Wirksamkeit eines erzeugten magnetischen oder elektromagnetischen Wechselfeldes kann entstehen infolge von z.B.
- Elektromagnetischer Einwirkung des Feldes auf die Ionenströme in einem Objekt, zum Beispiel einem menschlichen Körper (zum Beispiel Natrium- und Kaliumpumpen, Ca-Kaskade);
- Magnetomechanischer Einwirkung des Magnetfeldes auf die Schwingungsamplitude von Zellen und Organen (Resonanz);
- Ionisch-zyklotronischer Resonanz von Anionen und Kationen der Körperflüssigkeiten eines Objektes zur Verstärkung der Eigendreh-Impulse;
- Magnetischer Kernresonanz (Nuclear-Magnetic-Resonance (NMR));
- Elektronen-Spin-Resonanz (ESR).

Durch die elektromagnetische Wechselwirkung können daher vorteilhafte biophysiologische Effekte entstehen, z.B. bioelektrische Effekte, biochemische Effekte und bioenergetische Effekte. Der bioelektrische Effekt beispielsweise kann die Normalisierung einer Zellmembran verursachen. In pathologischen Fällen kann infolge des Eindringens von positiv geladenen Ionen, zum Beispiel von Na⁺ in das Zellinnere, das Membranpotential der Zelle fallen. Um diesen Prozess umzukehren, wird von der Zelle Energie benötigt, welche die Zelle aus der ATP-Hydrolyse schöpfen kann. Der biochemische Effekt beruht auf der Anhebung der Enzymaktivität, sowie der Aktivierung der oxydoreduktiven Prozesse, die mit ATP verbunden sind. Das wirksame Ion ist dabei das aus der Ca-Kaskade hergestellte Ca⁺⁺. Der bioenergetische Effekt stellt einen die Ernährung und das Zellwachstum stimulierenden Faktor dar. Ferner werden davon intrazelluläre Prozesse gesteuert, die zur Regeneration des Körpers führen. Solche Effekte können vorteilhaft durch Einbindung der Magnetfeldtherapie, z.B. durch Verwendung von Magneten, in verschiedenste Anwendungen erreicht werden.

Aus der CN 1130136 ist beispielsweise eine Vorrichtung bekannt, die Neodym-Magnete an Akupunkturstellen des Körpers mit leichtem oder festem Druck anpresst, um so die Akupunkturpunkte zu stimulieren. Diese Vorrichtung hat jedoch den Nachteil, dass der Druck der Magneten als störend empfunden werden kann. Außerdem besitzen diese Magnete an der Auflagefläche nur einen Pol, nämlich N oder S. Diese können jedoch konstruktiv bedingt kein Wechselfeld im Körper bilden.

Ein weiteres Beispiel hierzu zeigt die DE 103 41 958 A1 (Bayerische Motorenwerke AG, München). Die DE 103 41 958 A1 beschreibt Sitze für Kraftfahrzeuge unter Verwendung eines Permanentmagneten, wobei in der Rückenlehne des Sitzes mindestens ein beispielsweise flexibler Magnet angeordnet wird, um zahlreiche, sich abwechselnde magnetische Felder (im Folgenden magnetische Wechselfelder) zu erzeugen. Mit dieser Anordnung wird zwar die Wirkung der sogenannten pulsierenden Magnetfeldtherapie (elektrisch) imitiert, ein effektives pulsierendes Magnetfeld kann damit jedoch nicht aufgebaut werden. Zudem werden hier nur geringe Magnetfeldstärken erzielt.

Die DE 10 2004 058 722 A1 (Airbus Deutschland GmbH Deutschland GmbH, Hamburg) beschreibt einen Sitz mit einer Einrichtung zur Erzeugung einer elektromagnetischen Wechselwirkung, mit dem Ermüdungserscheinungen, wie sie beispielsweise auf Langstreckenflügen auftreten, vermieden werden können. Der Sitz der DE 10 2004 058 722 A1 verwendet dabei im Wesentlichen ein System zur Erzeugung eines Magnetfeldes, das auf dem Prinzip der elektro-Magnet-Resonanz-Stimulation (eMRS) beruht. Ein Beispiel dafür ist das eMRS^{®}-System der Firma vita-life^{®}. Die DE 10 2004 058 722 A1 beschreibt ferner noch eine Sitzbank, ein Fortbewegungsmittel, ein Magnetfeld-Resonanz-System unter Verwendung dr beschriebenen Einrichtung und die Verwendung einer solchen Einrichtung zur Erzeugung einer elektromagnetischen Wechselwirkung in einem Sitz oder in einer Sitzbank.

Leider zeigen die im Stand der Technik beschriebenen derartigen Vorrichtungen erhebliche Einflüsse durch die in den jeweiligen Sitzvorrichtungen verbauten Metallteile und Träger. Auch soll eine solche Vorrichtung periodische modulierte Magnetfelder erzeugen können. Zudem zeigen Vorrichtungen aus dem Stand der Technik zur Magnetresonanztherapie keine technische Umsetzung, die eine zuverlässige Stromerregung erlaubt. Es ist insbesondere von hohem Interesse, die Stromversorgung zu vereinfachen. So ist es bspw. wünschenswert, dass die Schaltung mit einer einzigen Gleichspannung versorgt wird und verschiedene Signalkonfigurationen beliebig erzeugt werden können. Bei medizinischen Geräten war es bislang erforderlich, ein separiertes, stabilisiertes Sicherheitsnetzgerät zu verwenden. Ein solches separiertes, stabilisiertes Sicherheitsnetzgerät ist jedoch kostenintensiv und erfordert einen Platzbedarf, der in den meisten Vorrichtungen nur schwer zu verwirklichen ist.

Im Stand der Technik werden im Zusammenhang mit der Stromerregung moderne Leistungsverstärker beschrieben, die es primär gestatten, eine bipolare Stromerregung, insbesondere bei dem Antrieb der Spulen von Lautsprechern und von analogen Instrumenten zu realisieren. Wie in der bekannten Fachliteratur ersichtlich ist, ermöglichen diese Vorrichtungen die Stromerregung im Tonfrequenzbereich und in niedrigem Frequenzbereich. Die üblichen Gegentaktverstärker, die im Allgemeinen mit Komplementärtransistoren aufgebaut sind, benötigen dabei eine symmetrische Versorgungsspannung. Bei den meistverbreiteten Lösungen wird im Gerät daher ein symmetrisches Netzteil oder ein Spannungswandler verwendet. Auch dies erfordert jedoch Platz, der u.U. kaum vorhanden ist.

Ein klassischer Verstärker der Betriebsart B, mit symmetrischer Stromversorgung wird z.B. im Buch von Patrick D. van der Puije (2002) "Telecommunication circuit design". John Wiley & Sons, Inc. N. Y. 161*. pi. be*schrieben. Diese Problematik der Stromversorgung, durch DC-DC Wandler mit hohem Wirkungsgrad, wurde auch im US Patent No 7009454 (2006) diskutiert.

Eine Brückenschaltung wird in Khandpur (2005): "Biomedical instrumentation". McGraw-Hill. New York. 132. p. geschrieben. In dieser Schaltung sind einerseits synchronisierte, separierte Eingangssignale mit umgekehrter Polarität auf zwei Seiten der Brücke erforderlich. Anderseits besteht keine Vollsymmetrie der Brücke.

Als vorteilhaft gilt auch eine DC-Signalübertragung, wobei die Symmetrie und die Eliminierung der Drift wichtig sind. Die oben angesprochenen Schaltungen sind jedoch nur begrenzt in der Lage, die mit beliebigen Hüllkurven modulierte, steile Impulsserien, in einem gesteuerten Schaltbetrieb mit niedriger Verzerrung zu erzeugen.

Der vorliegenden Erfindung lag daher die Aufgabe zugrunde, mit einer zweckmäßigen Konstruktion die oben erwähnten Nachteile zu eliminieren

Die Aufgabe wird durch die beigefügten Schutzansprüche gelöst.

Gemäß einer ersten Ausführungsform stellt die vorliegende Erfindung eine Sitz- oder Liegevorrichtung zur Erzeugung eines Magnetfeldes zur Magnetfeldtherapie bereit, umfassend
a) mindestens einen Aufnahmebereich;
b) eine Einrichtung, umfassend eine elektrische Leiterbahn, die zur bipolaren Stromerregung zur Erzeugung periodischer modulierter Magnetfelder im Aufnahmebereich eingerichtet ist;
c) eine aktive Brückenschaltung zur Steuerung der bipolaren Stromerregung zur Erzeugung periodischer modulierter Magnetfelder;
d) eine programmierbare Steuerung, die die Steuerung der Erzeugung periodischer modulierter Magnetfelder ermöglicht;
e) optional einen Sensor, der bevorzugt mit der Einrichtung nach b), der aktiven Brückenschaltung nach c) und/oder der Steuerung nach d) verbunden ist;
f) optional eine Energiequelle, die bevorzugt mit der der Einrichtung nach b), der aktiven Brückenschaltung nach c), der Steuerung nach d) und/oder dem Sensor nach e) verbunden ist.

Gemäß einer weiteren Ausführungsform stellt die vorliegende Erfindung eine Sitz- oder Liegevorrichtung zur Erzeugung eines Magnetfeldes zur Magnetfeldtherapie bereit, umfassend
a) mindestens einen Aufnahmebereich;
b) eine Einrichtung, umfassend eine elektrische Leiterbahn, die zur bipolaren Stromerregung zur Erzeugung periodischer modulierter Magnetfelder im Aufnahmebereich eingerichtet ist;
c) optional eine aktive Brückenschaltung zur Steuerung der bipolaren Stromerregung zur Erzeugung periodischer modulierter Magnetfelder;
d) eine programmierbare Steuerung, die die Steuerung der Erzeugung periodischer modulierter Magnetfelder ermöglicht;
e) einen Sensor, der bevorzugt mit der Einrichtung nach b), der aktiven Brückenschaltung nach c) und/oder der Steuerung nach d) verbunden ist;
f) optional eine Energiequelle, die bevorzugt mit der der Einrichtung nach b), der aktiven Brückenschaltung nach c), der Steuerung nach d) und/oder dem Sensor nach e) verbunden ist.

Die Erfinder haben hierbei in zahlreichen Anwendungen den Erfolg eines Magnetfeldes zur Magnetfeldtherapie mit einer wie hier beschriebenen Technik, insbesondere mit einer wie hier beschriebenen aktiven Brückenschaltung zur Therapie und Entspannung gezeigt. Einige Auszüge der hierbei durchgeführten Experimente sind in den Beispielen gezeigt.

Die erfindungsgemäße Sitz- oder Liegevorrichtung ist typischerweise ein Sitz, eine Liege oder Matratze, oder eine Auflage für diese Sitz- oder Liegevorrichtungen. Beispielsweise kann die erfindungsgemäße Sitz- oder Liegevorrichtung ausgewählt werden aus einer Sitzvorrichtung, z.B. einem Autositz, insbesondere einem Autositz für Personenkraftwagen, Lastkraftwagen, Busse, und Nutzfahrzeuge, einem Flugzeugsitz, Flugbegleiter- bzw. Cockpitsitze, einem Sitzmöbel aus dem Wohnbereich oder Arbeitsbereich, z.B. einem Stuhl, einem Fernsehsessel, einem Couchsessel, einem Bürostuhl, oder einem ärztlichen Behandlungsstuhl, oder einer Auflage für diese Sitzvorrichtungen, etc. Alternativ kann die erfindungsgemäße Sitz- oder Liegevorrichtung ausgewählt werden aus einer Liege oder Matratze, z.B. einer Liege oder Matratze für den häuslichen Gebrauch, einer Liege oder Matratze für den ärztlichen oder therapeutischen Gebrauch, einer Krankenhausliege oder -matratze, einer Massageliege oder -matratze, oder einer Auflage für diese Liegevorrichtungen. Die erfindungsgemäße Sitz- oder Liegevorrichtung kann alternativ auch ein Rollstuhl sein.

Die erfindungsgemäße Sitz- oder Liegevorrichtung umfasst zunächst mindestens einen Aufnahmebereich, wobei dieser Aufnahmebereich bevorzugt zur Aufnahme einer Einrichtung zur Erzeugung einer elektromagnetischen Wechselwirkung gestaltet ist, z.B. einer Einrichtung, umfassend eine elektrische Leiterbahn, die zur bipolaren Stromerregung zur Erzeugung periodischer modulierter Magnetfelder geeignet ist. Noch stärker bevorzugt ist die Einrichtung zur Erzeugung einer elektromagnetischen Wechselwirkung in den Aufnahmebereich integriert

Die erfindungsgemäße Sitz- oder Liegevorrichtung kann je nach Erfordernis mindestens einen Aufnahmebereich umfassen, z.B. 1, 2, 3, 4, 5, 6, 7, 8, 9, 10, etc., Aufnahmebereiche, wobei diese Aufnahmebereiche an verschiedenen Stellen der erfindungsgemäßen Sitz- oder Liegevorrichtung enthalten sein können. Hierbei kann jedes hier beschriebene Element der erfindungsgemäßen Sitz- oder Liegevorrichtung getrennt in einem separaten Aufnahmebereich untergebracht sein oder zusammen mit mindestens einem weiteren Element. Solche Elemente werden im Folgenden als Einrichtung zur Erzeugung einer elektromagnetischen Wechselwirkung beschrieben.

Die Einrichtung zur Erzeugung einer elektromagnetischen Wechselwirkung kann eine elektrische Leiterbahn umfassen, die zur bipolaren Stromerregung zur Erzeugung periodischer modulierter Magnetfelder im Aufnahmebereich eingerichtet ist; eine aktive Brückenschaltung zur Steuerung der bipolaren Stromerregung zur Erzeugung periodischer modulierter Magnetfelder; eine programmierbare Steuerung, die die Steuerung der Erzeugung periodischer modulierter Magnetfelder ermöglicht; einen Sensor, der bevorzugt mit der Einrichtung nach b), der aktiven Brückenschaltung nach c) und/oder der Steuerung nach d) verbunden ist; und/oder eine Energiequelle, die bevorzugt mit einer oder mehreren dieser Einrichtungen verbunden sein kann.

Bevorzugt ist die Einrichtung zur Erzeugung einer elektromagnetischen Wechselwirkung derart gestaltet, dass sie eine elektromagnetische Wechselwirkung in dem mindestens einen Aufnahmebereich und/oder einem definierten Bereich um den mindestens einen Aufnahmebereich erzeugt. Dabei ist die Einrichtung zur Erzeugung einer elektromagnetischen Wechselwirkung bevorzugt an oder in dem Sitz befestigt, insbesondere integriert.

Der mindestens eine Aufnahmebereich kann so angeordnet sein, dass die zu behandelnden bzw. zu therapierenden Körperteile der darauf sitzenden oder liegenden Person abgedeckt werden. So kann bspw., falls es sich bei der erfindungsgemäßen Sitz- oder Liegevorrichtung um eine Sitzvorrichtung handelt, der Aufnahmebereich in der Fußstütze, dem Beinbereich, der Sitzfläche, der Rückenlehne und/oder der Kopfstütze der Sitzvorrichtung enthalten sein. Falls die erfindungsgemäße Sitz- oder Liegevorrichtung eine Liege oder Matratze ist, kann der Aufnahmebereich in der Fußfläche, dem Beinbereich, dem Hüftbereich, dem Rückenbereich und/oder dem Kopfbereich der Liege oder Matratze enthalten sein.

Der mindestens eine Aufnahmebereich kann ferner auch extern oder am Rande der erfindungsgemäßen Sitz- oder Liegevorrichtung angeordnet sein. Ein solcher wie zuvor beschriebene Aufnahmebereich kann bspw. eine Steuerungseinrichtung, ein Panel, ein Bedienfeld, eine Datenspeichervorrichtung und/oder eine (Benutzer-)Schnittstelle, z.B. für eine Datenspeichervorrichtung, oder andere hier beschriebene Komponenten, umfassen.

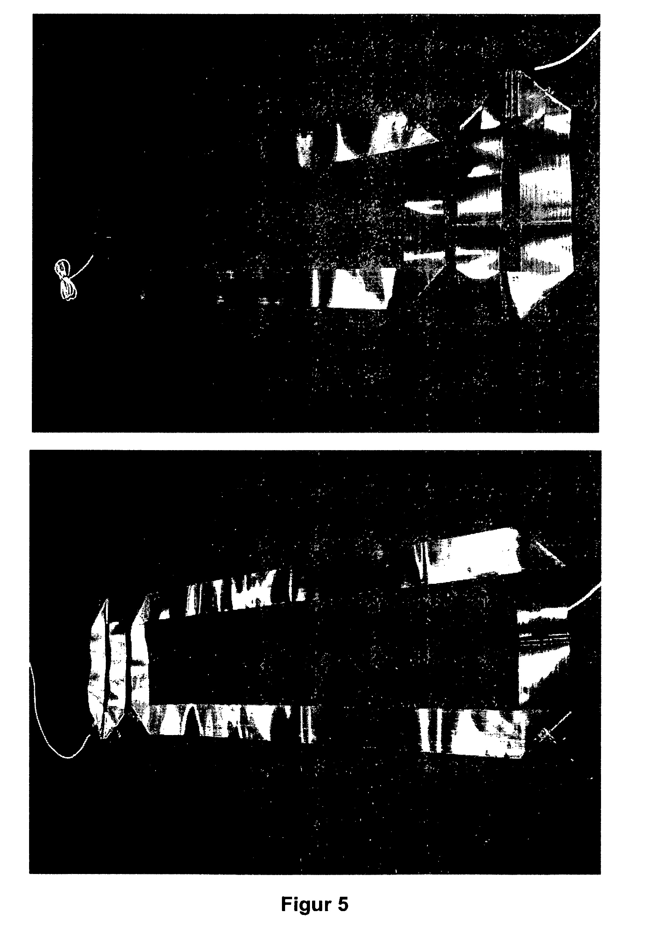

Die elektrische Leiterbahn der Einrichtung zur Erzeugung einer elektromagnetischen Wechselwirkung, die zur bipolaren Stromerregung zur Erzeugung periodischer modulierter Magnetfelder im Aufnahmebereich eingerichtet ist, kann ein mehradriges Flachkabel, eine Superlight-Spule, eine Superlight-Spule gefertigt aus mehradrigen Flachkabeln, einen Flexleiter, einen Flexleiter gefertigt aus mehradrigen Flachkabeln, oder eine daraus gefertigte Spule, oder eine sonstige geeignete Applikatorspule umfassen, die jeweils geeignet sind, durch bipolare Stromerregung ein periodisches moduliertes Magnetfelder zu erzeugen. Bevorzugt ist insbesondere ein mehradriges, sehr flaches und leichtes Flachkabel (s. z.B.: Fig. 5), wie es beispielsweise industriell eingesetzt wird, als Flächenspule/elektrische Leiterbahn. Die elektrische Leiterbahn der Einrichtung zur Erzeugung einer elektromagnetischen Wechselwirkung kann zusätzlich oder alternativ eine (Magnet-)Matte enthalten und/oder als (Magnet-)Matte ausgelegt sein und/oder eine oder einer Mehrzahl von bevorzugt wie oben beschriebenen Spulen zur Erzeugung einer elektromagnetischen Wechselwirkung enthalten. Bspw. kann eine solche (Magnet-)Matte eine oder mehrere Spulen umfassen, die eine der Matte angepasste Form hat. Diese Spule bzw. jede Spule der (Magnet-)Matte kann von der aktiven Brückenschaltung und/oder ggf. einem weiteren Steuergerat gesteuert werden. Die Spulen können dabei bspw. in Kreisen, Bogen, konzentrischen Ringen, quadratisch, rechteckig oder jeder sonstigen, z.B. der Flache angepasster, Form, etc., nebeneinander, ineinander oder auch übereinander angeordnet sein.

Ferner umfasst die erfindungsgemäße Sitz- oder Liegevorrichtung bevorzugt eine aktive Brückenschaltung zur Steuerung der bipolaren Stromerregung zur Erzeugung periodischer modulierter Magnetfelder. Die hier beschriebene aktive Brückenschaltung zur Steuerung der bipolaren Stromerregung dient zur Erzeugung periodischer modulierter Magnetfelder und ist typischerweise **dadurch gekennzeichnet, dass** zwei P-Kanal-MOSFET (1, 3), zwei N-Kanal-MOSFET (2, 4) und zwei Widerstande (15,20) in einer Brücke (B) integriert sind, die Quellen (S1, S3) von P-Kanal-MOSFET (1, 3) zu der positiven Speisespannung geschaltet sind, der Drain (D1) von P-Kanal-MOSFET (1) und der Drain (D2) von N-Kanal-MOSFET (2) gekoppelt sind, der Drain (D3) von P-Kanal-MOSFET (3) und der Drain (D4) von N-Kanal- MOSFET (4) zueinander geschaltet sind, zu den Ausgängen der Brückenschaltung (22, 23) eine Applikatorspule (21) gekoppelt ist, zwei Steuereinheiten (I, III) zu den P-Kanal-MOSFET (1, 3) und zwei Steuereinheiten (II, IV) zu den N-Kanal-MOSFET (2, 4) geschaltet sind, die Steuereinheiten (III, IV) durch das Kabel des Enable-Signals "A" (24) und die Steuereinheiten (I, II) durch das Kabel des Enable-Signals "B" (25) verbunden sind, das für die Eingangssignale vorgesehene Kabel (26) zu den Steuereinheiten (II, IV) gekoppelt ist und die Steuereinheiten (II, IV), sowie die Widerstände (15, 20) mit einem Erdungskontakt (GND) der Brücke (B) verbunden sind.

Besonders bevorzugt umfasst die erfindungsgemäße Sitz- oder Liegevorrichtung eine wie zuvor beschriebene aktive Brückenschaltung, wobei die aktive Brückenschaltung zur Steuerung der bipolaren Stromerregung zur Erzeugung periodischer modulierter Magnetfelder dadurch gekennzeichnet ist, dass der Eingangspunkt EN-B durch Widerstände (12, 13) zu Transistoren (5, 6) geschaltet ist, der Kollektor (K5) des Transistors (5) einerseits durch einen Widerstand (11) zur positiven Speisespannung (+), andererseits zu dem Tor (G1) des P-Kanal-MOSFET (1) gekoppelt ist, die Quellen (S1, S3) der P-Kanal-MOSFET (1, 3) zur positiven Speisespannung (+) geschaltet sind, der Eingangspunkt EN-A durch Widerstände (17, 18) zu Transistoren (7, 8) geschaltet ist, der Kollektor (K7) des Transistors (7) einerseits MOSFET (3) gekoppelt ist, der Eingang (A-input) durch Widerstände (14, 19) einerseits zu Kollektoren (K6, K8) von Transistoren (6, 8), andererseits zu nicht-invertierenden Eingängen (03, 05) von Operationsverstärkern (9, 10) geschaltet ist, die Ausgangspunkte (O1, O7) von Operationsverstärkern (9, 10) zu Toren (G2, G4) der N-Kanal-MOSFET (2,4) gekoppelt sind, die invertierenden Eingänge (02, 06) zu den Quellen (S2, S4) der N-Kanal- MOSFET (2, 4), sowie zu Widerständen (15, 20) geschaltet sind, die Emitter (E5, E6, E7, E8) der Transistoren (5, 6, 7, 8) und die Widerstände (15, 20) mit dem Erdungskontakt (GND) verbunden sind.

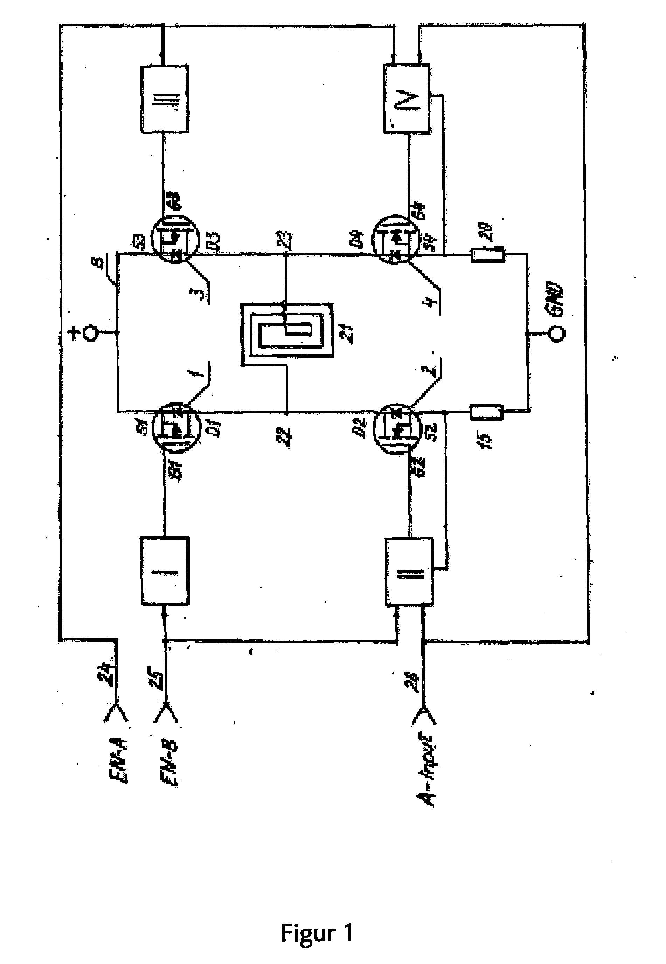

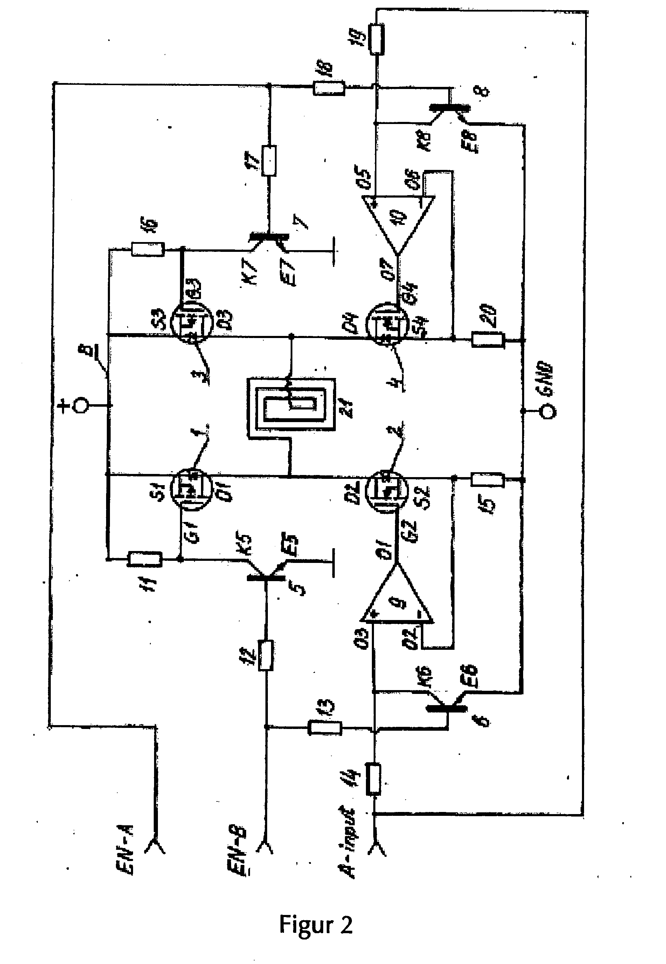

Die in der erfindungsgemäßen Sitz- oder Liegevorrichtung verwendete aktive Brückenschaltung ist bevorzugt eine aktive Brückenschaltung, wie in den Figuren 1, 2 und/oder 3 beschrieben.

Die Vorteile dieser aktiven Brückenschaltung bestehen in ihrer Steuerbarkeit und Wirtschaftlichkeit. Für die symmetrische Betriebsart braucht diese Schaltung nur eine einzige, positive Speisespannung und vereinfacht so effektiv das Stromversorgungsverfahren für die erfindungsgemäße Sitz- oder Liegevorrichtung. Ihre zweckmäßige Anordnung ermöglicht ferner die Steuerung der Applikatorspule mit zahlreichen Signalvariationen, insbesondere für eine sinus- und impulsförmige, bipolare Stromerregung, die eine besondere physiologische Wirksamkeit für die Magnetfeldbehandlung aufzeigen. Die Neuheit dieser Anordnung besteht noch in ihrer komplexen, analog- und digitalen Steuerung, sowie in ihrem eigentlichen "common-mode" Steuerungsbetrieb, der eine invertierende Stufe erspart und eine stabile Funktion sichert. Die mit der Erfindung erzielten Vorteile bestehen insbesondere darin, dass die vier Elemente einer für Analogsignalübertragung konstruierten, symmetrischen Brückenschaltung durch vier Steuerstufen analog und digital gleichzeitig gesteuert ist und durch eine, zu den Ausgängen der Brücke gekoppelte Applikatorspule, geeignete Magnetfelderzeugung realisiert ist.

Die erfindungsgemäße Sitz- oder Liegevorrichtung kann ferner eine programmierbare Steuerung zur Erzeugung periodischer modulierter Magnetfelder umfassen. Bevorzugt ist dies eine programmierbare Steuerung, die eine individuell programmierbare und/oder eine vorprogrammierte Einstellung mit Auswahl von verschiedenen Parametern ermöglicht. Beispielsweise kann eine solche (individuell) programmierbare und/oder vorprogrammierte Einstellung die Einstellung der Magnetfeldstärke, die Dauer der Behandlung, der Körpergröße, des Körpergewichts, der Dynamik der Magnetfeldstärke über die Dauer der Behandlung, Einstellungen zu der zu behandelnden Körperzone, die Tageszeit und davon abhängige Programmierungen und Einstellungen, etc., umfassen. Typischerweise steht die programmierbare Steuerung zur Erzeugung periodischer modulierter Magnetfelder mit mindestens einer der hier beschriebenen Komponenten der erfindungsgemäßen Sitz- oder Liegevorrichtung in Kontakt, z.B. der Einrichtung zur Erzeugung einer elektromagnetischen Wechselwirkung, der elektrischen Leiterbahn, die zur bipolaren Stromerregung zur Erzeugung periodischer modulierter Magnetfelder im Aufnahmebereich eingerichtet ist; einer aktiven Brückenschaltung zur Steuerung der bipolaren Stromerregung zur Erzeugung periodischer modulierter Magnetfelder; einer Steuerung, die eine programmierbare Erzeugung periodischer modulierter Magnetfelder ermöglicht; einem Sensor, der bevorzugt mit der Einrichtung nach b), der aktiven Brückenschaltung nach c) und/oder der Steuerung nach d) verbunden ist; und/oder einer Energiequelle, die bevorzugt mit einer oder mehreren dieser Einrichtungen verbunden sein kann. Die Steuerung kann elektronisch oder mechanisch erfolgen. Die Eingabe kann manuell, z.B. über eine Tastatur, oder über eine Sprachsteuerung erfolgen.

Gemäß einer bevorzugten Ausführungsform ist die programmierbare Steuerung zur Erzeugung periodischer modulierter Magnetfelder der erfindungsgemäßen Sitz- oder Liegevorrichtung intern an oder in der Sitz- oder Liegevorrichtung befestigt oder extern an der Sitz- oder Liegevorrichtung vorhanden.

Gemäß einer bevorzugten Ausführungsform ist die programmierbare Steuerung zur Erzeugung periodischer modulierter Magnetfelder der erfindungsgemäßen Sitz- oder Liegevorrichtung extern befestigt, z.B. über eine Fernbedienung, über eine in einem Fortbewegungsmittel, z.B. Auto oder Flugzeug, enthaltenen Konsole, (elektronischen) Steuerung, oder Bordsystem oder ist darin integriert. Besonders bevorzugt wird die programmierbare Steuerung, z.B. in automotiven Anwendungen, in das bestehende Bordsystem integriert, oder z.B. über eine an der erfindungsgemäßen Sitz- oder Liegevorrichtung befestigten Konsole. So kann z.B. die Integration in ein Rahmenteil, etc., der erfindungsgemäßen Sitz- oder Liegevorrichtung erfolgen, bspw. als Fernbedienung. Dies garantiert die Kompaktheit des Gesamtsystems.

Zusätzlich kann die programmierbare Steuerung zur Erzeugung periodischer modulierter Magnetfelder der erfindungsgemäßen Sitz- oder Liegevorrichtung oder die erfindungsgemäße Sitz- oder Liegevorrichtung selbst eine Benutzerschnittstelle umfassen, die zum benutzerdefinierten Einstellen der elektromagnetischen Wechselwirkung eingerichtet ist. Diese Benutzerschnittstelle kann in die programmierbare Steuerung zur Erzeugung periodischer modulierter Magnetfelder integriert sein, eine eigenständige Komponente darstellen oder aber unabhängig von der Steuerung in der erfindungsgemäßen Sitz- oder Liegevorrichtung vorkommen. Vorteilhaft wird mittels einer solchen Benutzerschnittstelle (zum Beispiel eine Art Fernbedienung, eine Datenschnittstelle, wie z.B. eine USB-Schnittstelle, eine serielle oder parallele Schnittstelle, etc., oder ein Datenspeicher) ein Benutzer in die Lage versetzt, individuelle Einstellungen der elektromagnetischen Wechselwirkung vorzunehmen. Der Benutzer kann die Wirkung somit an seine individuellen Bedürfnisse anpassen. Auch kann jemand Drittes, z.B. ein Hersteller, Unternehmen, ein betreuender Arzt oder Betreuer des Systems, etc. Vorprogrammierungen durchführen, z.B. die Einstellung der Magnetfeldstärke, die Dauer der Behandlung, der Körpergröße, des Körpergewichts, der Dynamik der Magnetfeldstärke über die Dauer der Behandlung, das zu behandelnde Körperteils oder die zu behandelnde Körperzone, Einstellungen zu dem zu behandelnden Körperteil oder der Körperzone, die Tageszeit und davon abhängige Programmierungen und Einstellungen, etc.

So kann bspw. über ein Programm eine unterschiedliche Magnetfeldstärke für die verschiedenen Tageszeiten eingegeben werden, z.B. Nachts zum Schlafen, Morgens zum Aufwachen (Aktivieren), Tagsüber zum Aktivieren oder Entspannen, und Abends zum Entspannen, etc. Es kann ferner ein frei wählbares Programm eingegeben werden, z.B. ein Wellness-Programm, Fitnessprogramm, das bevorzugt für beispielsweise eine stärkere Durchblutung sorgen könnte, oder ein Programm wie zuvor beschrieben.

Vorteilhaft kann eine solche Benutzerschnittstelle, die zur Einstellung der Parameter für eine elektromagnetische Wechselwirkung vorgesehen ist, in ein bereits vorhandenes Bedienterminal, Entertainment-System oder Steuerungssystem integriert sein. Eine Benutzerschnittstelle könnte auch eine Einrichtung zum Aufnehmen einer Chip-Karte oder eines sonstigen Speichermediums aufweisen, auf der ein Programmcode mit Anweisungen für die Einstellungen gespeichert ist und diesen Code auf das Steuergerät laden, von wo es ausgeführt werden kann. Austauschbare Chip-Karten oder Speichermedien haben den Vorteil, dass jederzeit neue Eigenschaften und Verbesserungen des Systems genutzt werden können und dass die Funktionalität auf einen Benutzer optimal angepasst werden kann (zum Beispiel auf Körpermaße, Geschlecht, bekannte Krankheiten, etc.). Zum Beispiel könnte eine Chip-Karte oder Speichermedium speziell für wie oben definiertes Programm vorgesehen sein. Eine Einrichtung zur Erzeugung einer elektromagnetischen Wechselwirkung mit einem solchen Programm, z.B. auf einer solchen Chip-Karte oder Speichermedium, hätte das Ziel, eine besondere Wohlfühlsituation für einen Benutzer zu schaffen.

Gemäß einer weiteren Ausführungsform weist die erfindungsgemäße Sitz- oder Liegevorrichtung eine Sensoreinrichtung auf, die zum Erfassen mindestens eines physiologischen Parameters eines Nutzers ausgestaltet ist. Mittels des gewonnenen physiologischen Parameters kann die Einrichtung zur Erzeugung einer elektromagnetischen Wechselwirkung steuerbar sein. Eine Sensoreinrichtung könnte beispielsweise ein Biofeedback-Sensor zur Ermittlung des aktuellen Körperzustandes eines Nutzers des Sitzes oder der Liegevorrichtung sein. Die Signale, die von dem Körper des Anwenders gewonnen werden, können an den Sitz oder der Liegevorrichtung, insbesondere an eine Steuereinheit weitergegeben werden, z.B. eine wie hier beschriebene Steuerung. Diese Steuereinheit kann die elektromagnetische Wechselwirkung (beispielsweise eine elektrische, magnetische oder elektromagnetische Feldstärke) entsprechend dem Zustand des Körpers des Objektes einstellen. Beispiele für Messgrößen, die als Biofeedback-Signale genutzt werden können, wäre eine Hautwiderstandsmessung, ein Elektromyogramm (EMG), eine Messung der Hauttemperatur, ein Elektroenzephalogramm (EEG), eine Sauerstoffpartikel-Druckmessung oder eine Bestimmung der Herzratenvariabilität (HRV).In diesem Zusammenhang bedeutet Biofeedback bevorzugt die Rückleitung von Messdaten zum Patienten, um seine unbewussten Körperfunktionen zu beeinflussen. Die Rückleitung der Messdaten muss nicht notwendig an den Patienten erfolgen, sondern kann an entweder an die Steuerung oder Therapiegeräte erfolgen, die durch die so erhaltenen Informationen den Verlauf und die Dosis der Therapie automatisch so anpassen können, dass die maximale Wirkung erreicht wird. Mittels Biofeedback-Komponenten kann dann bspw. eine automatische Steuerung des Behandlungsablaufs erfolgen. Ein Beispiel eines Biofeedback-Sensors bzw. einer Sensoreinrichtung wäre ein Fingersensor, mit dem die Varianz der Herzschläge (HRV) laufend gemessen wird. Die Messdaten bewirken in Echtzeit eine laufende Anpassung der Dosis, beispielsweise eines elektrischen, magnetischen oder elektromagnetischen Feldes. Die HRV als energetische Diagnosemethode kann interessant sein, weil sie einen Parameter misst, der über den gesamten Organismus eines Objektes Auskunft gibt und nicht nur eine Momentanaufnahme an einem bestimmten Punkt liefert. Der Fingersensor kann an den Finger eines Objektes bzw. sitzenden Nutzers angeschlossen werden und liefert eine Rückmeldung, die zur Dosisfindung verwendet werden kann. Ein weiteres Beispiel eines Biofeedback-Sensors bzw. einer Sensoreinrichtung wäre ein Handgelenks- oder Brustband zur Puls und Herzfrequenzmessung. Vorteilhaft ermöglicht der Einsatz eines Biofeedback-Sensors bzw. einer Sensoreinrichtung eine effektive und benutzerspezifische Möglichkeit, eine elektromagnetische. Wechselwirkung zu justieren.

Gemäß einem weiteren Ausführungsbeispiel der vorliegenden Erfindung wird eine Anzeigeeinheit für die erfindungsgemäße Sitz- oder Liegevorrichtung bereitgestellt. Diese Anzeige kann ein Teil des oben beschriebenen Steuerungsgerätes sein. Eine solche Anzeigeeinheit kann derart ausgestaltet sein, dass sie einem in dem Aufnahmebereich angeordneten Objekt eine Anzeige über die einzustellenden Parameter, die aktuelle Behandlung, besondere Hinweise, etc. anzeigen kann. Diese Anzeige kann eine mechanische oder elektronische Anzeige sein, sie kann optische und/oder akustische Signale umfassen. Sie kann weiterhin eine sprachgesteuerte Benutzerführung beinhalten.

Bei dem mit der erfindungsgemäßen Sitz- oder Liegevorrichtung erzeugten Magnetfeld handelt es sich primär um eine elektromagnetische Wechselwirkung. Dieses Feld kann statisch, homogen oder pulsierend mit einer gezielt vorgebbaren Impulsform sein, insbesondere pulsierend. Die Impulsform kann aus der Gruppe einer Dreiecksform, einer Rechteckform, einer Sägezahnform, einer inversen Sägezahnform, einer sinusförmigen Kurve oder einer Hüllkurve ausgewählt sein, besonders bevorzugt aus einer sinusförmigen Kurve oder einer Hüllkurve. Eine Hüllkurve ist dabei typischerweise eine Kurve, die jede Kurve einer Kurvenschar in einem Punkt berührt. Eine Hüllkurve kann dabei auch die Hüllkurve eines Frequenzspektrums sein und verbindet die Spitzen der Frequenzlinien. Unter einer Sägezahnform versteht man insbesondere eine Mehrfach-Sägezahn Impulsform oder ein Hüllkurvensignal, die eine Überlagerung einer Mehrzahl von Sägezahnformen darstellt. Dabei können die Frequenz bzw. die magnetische Flussdichte variiert werden, um bestimmte Effekte zu erreichen. Die praktische Erfahrung hat gezeigt, dass pulsierende Magnetfeldsysteme vorteilhafter sind als statische Magnetfelder. Bevorzugt werden pulsierende elektromagnetische Feldstärken mit maximalen Flussdichten der Applikator-Abstrahlungen von 1 bis 10.000 µT, bevorzugt 10-800 µT, noch stärker bevorzugt 10-600 µT und am stärksten bevorzugt zwischen 10 und 100 µT erzeugt und erfindungsgemäß eingesetzt.

Beispielsweise kann die Frequenz bzw. die magnetische Flussdichte variiert werden, um bestimmte Effekte, wie bspw. Entspannung, Aktivierung, Behandlung von Schmerz, Behandlung von Gelenken, etc. zu erreichen. So kann bspw. zur Entspannung eine (pulsierende) elektromagnetische Feldstärke mit maximalen Flussdichten der Applikator-Abstrahlungen von etwa 10 bis 40 µT verwendet werden, zur Aktivierung eine (pulsierende) elektromagnetische Feldstärke mit maximalen Flussdichten der Applikator-Abstrahlungen von etwa 30 bis 100 µT, zur Behandlung von Schmerz eine (pulsierende) elektromagnetische Feldstärke mit maximalen Flussdichten der Applikator-Abstrahlungen von etwa 10 bis 800 µT, zur Behandlung von Gelenken eine (pulsierende) elektromagnetische Feldstärke mit maximalen Flussdichten der Applikator-Abstrahlungen von etwa 10 bis 10.000 µT, alle vorgenannten Feldstärken bevorzugt als pulsierende elektromagnetische Feldstärke, und/oder mit sinusförmigen -, Rechteck-, Sägezahn -, inversen Sägezahnimpulsen oder Frequenzbändern, etc., noch stärker bevorzugt mit Hüllkurven aus diesen Signalen, Impulsen oder Frequenzbändern, am stärksten bevorzugt in einem Frequenzbereich zwischen etwa 1 und 300 Hz, etc.

Für die Magnetfeldtherapie werden vorzugsweise sogenannte extremity low frequency electromagnetic fields (ELF-EMF) genutzt, deren elektrische Parameter kleiner oder gleich denen des Erdmagnetfelds sind, während die Intensität ihres magnetischen Feldes größer oder gleich der des Erdmagneten ist. Dabei können externe magnetische Felder auf die in biologischen Systemen vorliegenden geladenen Teilchen wirken. Sie können diese ablenken (Lorentz-Kraft, Hall-Effekt) und ihre Strahlung fokussieren.

Magnetische Felder der Frequenz 20 Hz beeinflussen die Zellmembran-Permeabilität von Ca-Ionen (Cyclotronen-Resonanz). Neben einer solchen Beeinflussung des lonenflusses können magnetische Felder paramagnetische Teilchen etwa in Coenzymen oder prosthetischen Gruppen (zum Beispiel das Eisenzentrum in der Hämebene von Hämoglobin oder Myoglobin) und damit die Enzymaktivität beeinflussen. Sie wirken auf Flüssigkristalle ein und beeinflussen so Membranstrukturen. Solche Magnetfeldstärken der Frequenz 20 Hz sind daher ggf. bevorzugt.

Gemäß einer anderen bevorzugten Ausführungsform umfasst die erfindungsgemäße Sitzoder Liegevorrichtung einen Sensor. Dieser Sensor dient bevorzugt dazu, festzustellen, ob die erfindungsgemäße Sitz- oder Liegevorrichtung gerade in Benutzung ist oder nicht und ob die Magnetfeldtherapie erfolgen soll oder kann. Ein solcher Sensor erlaubt damit die automatisierte Steuerung der Magnetfeldtherapie im Zusammenhang mit der erfindungsgemäßen Sitz- oder Liegevorrichtung. Dies betrifft sowohl das automatisierte Einschalten als auch das automatisierte Ausschalten der Magnetfeldtherapie über eine der Komponenten der erfindungsgemäßen Sitz- oder Liegevorrichtung. So kann die erfindungsgemäße Sitzoder Liegevorrichtung so gestaltet sein, dass der Sensor die bipolare Stromerregung zur Erzeugung periodischer modulierter Magnetfelder bei Benutzung anschaltet und bei NichtBenutzung wieder ausschaltet. Durch das automatisierte Einschalten kann bspw. je nach Tageszeit, je nach Gewicht und/oder je nach individueller Einstellung ein vorprogrammiertes Magnetfeld erzeugt werden. Durch das automatisierte Ausschalten kann einem unerwünschten Energieverbrauch entgegengewirkt werden, wenn das Gerät ohne Inanspruchnahme durch einen Benutzer läuft. Der Sensor kann mit jeder der oben genannten Komponenten verbunden sein, z.B. der Einrichtung zur Erzeugung einer elektromagnetischen Wechselwirkung, der elektrischen Leiterbahn, der aktiven Brückenschaltung zur Steuerung der bipolaren Stromerregung zur Erzeugung periodischer modulierter Magnetfelder; der Steuerung, die eine programmierbare Erzeugung periodischer modulierter Magnetfelder ermöglicht; und/oder der Energiequelle. Der Sensor kann ein Erkennungssensor im Allgemeinen sein, bspw. ein Drucksensor, ein Infrarotsensor, ein Ultraschallsensor, ein optischer Sensor, ein optischer Sensor, der bspw. mit einer Lichtschranke verbunden ist, ein elektrischer Kontakt- oder Nähesensor, oder einem Sensor, der auf Veränderung des Dipolmomentes oder des Widerstandes reagiert, etc. Ein Drucksensor kann bspw. so gestaltet sein, dass der Drucksensor die bipolare Stromerregung zur Erzeugung periodischer modulierter Magnetfelder bei Belastung anschaltet und bei Entlastung wieder ausschaltet, z.B. durch Erkennung eines Mindestdruckes oder -gewichtes, der/das als Auslöseschwelle dient. Ein Infrarotsensor kann z.B. bei Überschreitung einer Mindesttemperatur, z.B. einer Temperatur oberhalb der Raumtemperatur, z.B. einer Temperatur von mehr als 25°C, bevorzugt in einem Bereich von etwa 25°C bis etwa 40°C, bevorzugt bei etwa 37°C, die bipolare Stromerregung zur Erzeugung periodischer modulierter Magnetfelder aktivieren. Ein Ultraschallsensor, ein optischer Sensor, z.B. ein optischer Sensor, der bspw. mit einer Lichtschranke verbunden ist, ein elektrischer Kontakt- oder Nähesensor, etc., kann bspw. so gestaltet sein, dass der Sensor die bipolare Stromerregung zur Erzeugung periodischer modulierter Magnetfelder bei Annäherung einer Person ab einem bestimmten Abstand einschaltet und bei dieser Person oder des Objekts Entfernung wieder ausschaltet. Ebenso kann bei einem Sensor, der auf Veränderung des Dipolmomentes oder des Widerstandes reagiert, eine Veränderung des elektrischen Feldes oder Widerstandes die bipolare Stromerregung zur Erzeugung periodischer modulierter Magnetfelder aktivieren. Weitere Sensoren können, je nach Bedarf eingesetzt werden. Dabei kann jegliche Sensorik eingesetzt werden, die es ermöglicht festzustellen, ob sich eine Person auf der erfindungsgemäßen Sitz- oder Liegevorrichtung befindet. Eine Kombination der zuvor genannten Sensoren ist ebenfalls möglich.

Gemäß einer weiteren bevorzugten Ausführungsform umfasst die erfindungsgemäße Sitzoder Liegevorrichtung eine Energiequelle, wobei die Energiequelle extern oder intern vorliegt. Die Energiequelle kann einen herkömmlichen Gleich- oder Wechselstrom umfassen bzw. eine Energiequelle, die einen herkömmlichen Gleich- oder Wechselstrom erzeugt, z.B. 12V, 14V, 42V, 110V, 220 V, etc. Dies kann bspw. das Bordnetz eines Fortbewegungsmittels sein, z.B. eines Autos, eines Flugzeugs, eines Schiffs, oder eine herkömmliche an ein Stromnetz angeschlossene Steckdose, z.B. in einem Haushalt oder einer Büroeinrichtung oder einer medizinischen Einrichtung, z.B. einem Krankenhaus.

Die erfindungsgemäße Sitz- oder Liegevorrichtung kann in verschiedenen Bereichen eingesetzt werden. Bevorzugte Anwendungsbereiche sind dabei vor allem die Anwendung in Matratzen (Matratzenapplikation), für Privatanwender, bei Kassen- und Privatpatienten in der Klinik, in Alters,- und Pflegeheimen, bei Kur- und Wellnesseinrichtungen, im Bereich Luftfahrt (Aircraft), insbesondere dort im geschäftlichen Bereich, bei Schiffsreisen, bei Thrombosepatienten, zur Jetlag-Prophylaxe, zum Lösen von Verspannungen durch langes Sitzen, im Eisenbahnbereich, insbesondere dort im geschäftlichen Bereich, zum Lösen von Verspannungen durch das lange Sitzen, bei Automotive-Anwendungen, zur Steigerung der Konzentration und Aufmerksamkeit, zum Lösen von Verspannungen bei längeren Autofahrten, im Bürobereich (Office), zur Regeneration und Stressabbau, zur Erhöhung der Leistungsbereitschaft, zur Erhöhung des allgemeine Wohlgefühls und somit der Motivation, und zur Erhöhung der Konzentrationsfähigkeit.

Gemäß einer Ausführungsform kann die Erfindung, z.B. bei Matratzen eingesetzt werden. Bevorzugt wird dabei eine vollautomatisierte Anwendung, die für den Endkunden einen besonderen Vorteil darstellt.

Während des Schlafes sind vor allem zwei Phasen wichtig:
- Einschlafphase als Beginn einer vertieften Regeneration des Organismus
- Aktivierung am Morgen für eine erhöhte Leistungsbereitschaft

Für beide Phasen können unterschiedliche Magnetfeldstärken eingesetzt werden. Dies bewirkt einen umfassenden Stressabbau, löst Verspannungen, ist eine Prophylaxe für einige andere, durch gestörten Schlaf verursachte Krankheiten und steigert ganz allgemein das Wohlbefinden und die Leistungsfähigkeit des Organismus. Durch den Einbau eines Sensors oder einer entsprechenden Sensorik ist es möglich, zu erkennen, ob sich jemand auf der Matratze befindet. Ist die länger als eine vorbestimmte Zeit, so wird automatisch das Regenerationsprogramm gestartet. In Kombination mit einer Weckfunktion ist es am Morgen möglich, noch bevor die Person die Matratze verlässt, das Aktivierungsprogramm zeitgemäß zu starten. Der Ablauf kann damit vollautomatisiert werden. Dem Nutzer wird dadurch die Handhabung erheblich vereinfacht und Fehler in der Anwendung werden vermieden. Im Klinik,- und Altenpflegebereich erspart diese vollautomatisierte Applikation darüber hinaus den Aufwand für das Pflegepersonal, die Technik für die einzelnen Patienten bedienen zu müssen. Bei der Anwendung bei Matratzen ergibt sich neben den Privatanwendern, die bevorzugt zweimal täglich die Anwendung zur Regeneration und Aktivierung nutzen wollen, ein Vorteil auch für solche Patienten im Krankenhausbereich, bei denen die erfindungsgemäße Technik in die Klinikmatratze integriert wird. Bei Privatanwendern ist dabei eine einfache Bedienbarkeit bis hin zur Vollautomatisierung vorteilhaft. Bei Privatanwendern, bei Klinikpatienten, bei Patienten in Alters,- und Pflegeheimen, etc., erlaubt dies eine therapeutische Maßnahme, die in verschiedensten Ausprägungen verfügbar ist, wie etwa zur Wundheilung, Schmerztherapie, etc. Beispielsweise leiden Patienten in Alters,- und Pflegeheimen leider sehr oft an Dekubitus und allgemein an verschiedenen Schmerzzuständen. Hier setzt die direkt in die Matratze integrierte Magnetfeldtherapie dort an, wo sich diese Menschen am längsten befinden und wo auch der Ursprung für diese Schmerzzuständen lokalisiert ist. Bei Besuchern von Kur,- und Wellness-Einrichtungen sind diese Behandlungen eher temporärer Natur, wobei auch für diese Patienten entweder kurzfristig oder bei häufigeren Besuchen ein Therapieeffekt zu verzeichnen ist

Im Bereich der Luftfahrt liegt eine weitere bevorzugte Anwendung. Insbesondere bei Interkontinental - Flügen besteht die Gefahr von Thrombosen, Verspannungen durch das lange Sitzen und negative Auswirkungen durch den Jetlag allgemein. Bis dato gibt es jedoch trotz optimierter Sitze keine wirksamen Mittel in den Flugzeugsitzen, um dies wirksam bekämpfen zu können. Die Integration eines Magnetfeld-Resonanz-Systems entsprechend der vorliegenden Erfindung in einen Passagier-, Flugbegleiter- oder Cockpitsitz kann dabei zur Minderung des Jetlags sowie zur Minderung der Thrombosegefahr und zur Steigerung des allgemeinen Wohlbefindens des Reisenden durch die Förderung von Entspannung, Schlaf und Stressabbau beitragen.

Ein weiterer bevorzugter Anwendungsbereich stellt die Eisenbahn und deren Transportsysteme und Sitzgelegenheiten darin dar. Ähnlich wie in der Luftfahrt gibt es mittlerweile auch hier sehr umfangreich ausgestatte Business-Klassen vor allem in den Intercity-Zügen und eine immer stetig ansteigende Zahl an Reisenden mit entsprechenden Beschwerden nach einer langen Reise.

Ein anderer bevorzugter Anwendungsbereich stellt die Automobilindustrie und die Implementierung in Autos und Autositze dar. Dabei ist das Auto ist ein Ort vor allem für berufstätige Personen, in dem viel Zeit verbracht wird. Dabei ist gerade für den Fahrer Konzentration und Aufmerksamkeit erforderlich. Für die Passagiere ist oft dies ein Ort auch zur Regeneration und Stressabbau. Massagesitze, Belüftungen und Lordosenstützen erlauben bereits eine Entlastung, wobei die Implementierung der erfindungsgemäßen Technologie in solche Autositze einen deutlichen Beitrag zur Regeneration und Stressabbau leistet.

Andere Anwendungsbereiche umfassen bspw. Fitness-Center, Büro-Räume, etc. Gerade in Unternehmen stellt die erfindungsgemäße Technologie ein Vorteil dar. Durch Regeneration und Stressabbau während des Tagesgeschäfts werden Konzentration und Leistungsbereitschaft gefördert. Das allgemeine Wohlbefinden und das Lösen von Verspannungen trägt zu einer viel höheren Motivation und somit Qualität der Tätigkeiten bei. Ferner erlaubt dies, die Krankenstandstage in den Unternehmen zu reduzieren.

Ein weiterer relevanter Anwendungsbereich ist der Einsatz in Rollstühlen, wo Effekte der erzwungenen Immobilität des Betroffenen durch den Einsatz der erfindungsgemäßen Sitz- oder Liegevorrichtung gelindert werden können.

Ein separater Aspekt der Erfindung kann dabei auch allgemein ein Rollstuhl mit einer Vorrichtung zum Erzeugen eines Magnetfeldes zur Magnetfeldtherapie sein. Dabei ist die Vorrichtung zur Erzeugung des Magnetfeldes vorzugsweise - aber nicht zwangsläufig - so ausgeführt, wie in der erfindungsgemäßen Sitz- oder Liegevorrichtung. Der Rollstuhl dieses separaten Efindungsaspekts kann daher jede Art von Vorrichtung zur Erzeugung eines Magnetfeldes umfassen. Der Rollstuhl dieses separaten Erfindungsaspekts kann daher als Vorrichtung zur Erzeugung eines Magnetfeldes beispielsweise auch Permanantmagneten und/oder eine andere Art von Schaltung umfassen, solange diese Vorrichtung ein Magnetfeld erzeugt, vorzugsweise geeignet zur Magnetfeldtherapie.

### Figuren:

Die folgenden Figuren sollen die oben beschriebenen Gegenstände illustrieren und sind in keiner Weise einschränkend für den Gegenstand der Schutzansprüche. Ein Ausführungsbeispiel der in der Erfindung optional verwendeten aktiven Brückenschaltung ist in den Zeichnungen dargestellt und wird im Folgenden näher beschrieben.
- **Fig. 1:**: zeigt das Blockdiagramm der aktiven Brückenschaltung, die unter Verwendung von zwei P-Kanal-MOSFET 1, 3, zwei N-Kanal-MOSFET 2, 4 und zwei Widerständen 15, 20 aufgebaut ist. Die Quellen S1, S3 von P-Kanal-MOSFET 1, 3 sind zu der positiven Speisespannung der Brücke B geschaltet. Der Drain D1 von P-Kanal-MOSFET 1 und der Drain D2 von N-Kanal-MOSFET 2 sind gekoppelt. Der Drain D3 von P-Kanal- MOSFET 3 und der Drain D4 von N-Kanal-MOSFET 4 sind zueinander geschaltet. Eine Applikatorspule 21 ist zu den Ausgängen 22, 23 der Brücke B gekoppelt. Zwei Steuereinheiten I, III sind zu den P-Kanal-MOSFET 1, 3 und zwei Steuereinheiten II, IV sind zu den N-Kanal-MOSFET 2, 4 geschaltet. Die Steuereinheiten III, IV sind durch das Kabel 24 des Eingangs EN-A verbunden und die Steuereinheiten I, II sind durch das Kabel 25 des Eingangs EN-B 25 zusammengeschaltet. Der Eingang A- input ist durch das Kabel 26 zu den Steuereinheiten II, IV gekoppelt und die Steuer- einheiten II, IV sowie die Widerstände 15, 20 sind mit dem Erdungskontakt GND der Brücke B verbunden.

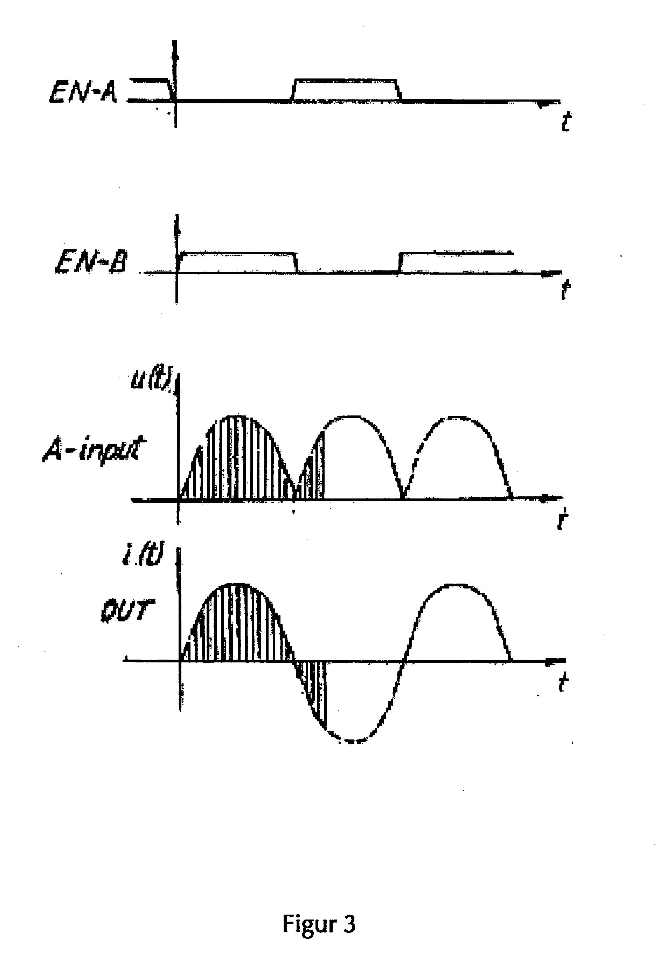

In Fig. 1 sind vier MOSFET 1, 2, 3, 4, abgebildet, die die aktive Brücke bilden. Die digitalen Signale, die laut der Fig. 3 zu den Eingängen EN-A und EN-B geschaltet sind, steuern die vier Steuereinheiten I, II, III, IV. Die Steuereinheiten I und III haben reine Schaltungsfunktion, abwechslungsweise öffnen und schließen die MOSFET 1 und 3 ab. Die Steuereinheiten II und IV haben eine komplexe Funktion. Diese Steu- ereinheiten II und IV empfangen die digitalen Steuersignale von den Eingängen EN- A, EN-B und gleichzeitig die durch Hüllkurven modulierten Signalserien von dem Eingang A-input. Nach der Verstärkung dieser modulierten Signale wird das Magnet- feld der vom Strom durchflossenen Applikatorspule 21 für die Behandlung zustande gebracht.
- **Fig. 2:**: zeigt eine Gesamtdarstellung der aktiven Brückenschaltung und stellt die aktive Brü- ckenschaltung ausführlich dar. Der Eingang EN-B ist durch Widerstände 12, 13 zu zwei Transistoren 5, 6 geschaltet. Der Kollektor K5 des Transistors 5 ist durch einen Widerstand 11 zur positiven Speisespannung (+) und zu dem Tor G1 des P-Kanal- MOSFET 1 gekoppelt. Die Quellen S1, S3 der P-Kanal-MOSFET 1, 3 sind zur positi- ven Speisespannung der Brücke B geschaltet. Durch Widerstände 17, 18 ist der Ein- gang EN-A zu zwei Transistoren 7, 8 geschaltet. Der Kollektor K7 des Transistors 7 ist einerseits durch einen Widerstand 16 zur positiven Speisespannung (+), anderer- seits zu dem Tor G3 des P-Kanal-MOSFET 3 gekoppelt. Der Eingang A-input ist durch Widerstände 14, 19 einerseits zu Kollektoren K6, K8 von Transistoren 6, 8, andererseits zu nicht invertierenden Eingängen 03, O5 von Operationsverstärkern 9, 10 geschaltet. Die Ausgangspunkte 01, 07 von Operationsverstärkern 9, 10 sind zu Toren G2, G4 der N-Kanal-MOSFET 2, 4 gekoppelt. Die invertierenden Eingänge 02, 06 von Operationsverstärkern 9, 10 sind zu den Quellen S2, S4 der N-Kanal- MOSFET 2, 4, sowie zu Widerständen 15, 20 geschaltet. Die Emitter E5, E6, E7, E8 der Transistoren 5, 6, 7, 8 und die Widerstände 15, 20 sind mit dem Erdungskontakt GND verbunden.
In Fig. 2 ist die Anordnung der aktiven Brückenschaltung ausführlich dargestellt. Die digitalen Signale, die laut Fig. 3 zu den Eingängen EN-A und EN-B geschaltet sind, steuern die vier Transistoren 5, 6, 7, 8, die Schaltfunktionen verrichten. Falls die Spannung am Eingangspunkt EN-A auf dem Pegel "0" ist und EN-B auf dem Pegel "1 ", dann sind die Transistoren 5 und 6 in Sättigung und die Transistoren 7 und 8 ab- geschlossen. In diesem Fall ist der P-Kanal-MOSFET 1 geöffnet, der N-Kanal- MOSFET 2 empfängt kein Signal vom Eingang A-input und ist abgeschlossen. Gleichzeitig ist der P-Kanal-MOSFET 3 auch abgeschlossen. Der N-Kanal-MOSFET 4 empfängt die modulierten Signalserien vom Eingang A-input und wird gleichzeitig geöffnet und gesteuert. Falls der Eingang EN-A auf dem Pegel "1" ist und EN-B auf dem Pegel "0", dann sind die Transistoren 7 und 8 in Sättigung, die Transistoren 5 und 6 abgeschlossen und die Polarität der Spannung auf der Applikatorspule 21 ist umgekehrt.
- **Fig. 3:**: zeigt Diagramme von Steuersignalen, Eingangs- und Ausgangssignalen, wie sie mit der aktiven Brückenschaltung gemäß Figur 1 und 2 erzeugt werden kön- nen/verwendet werden.

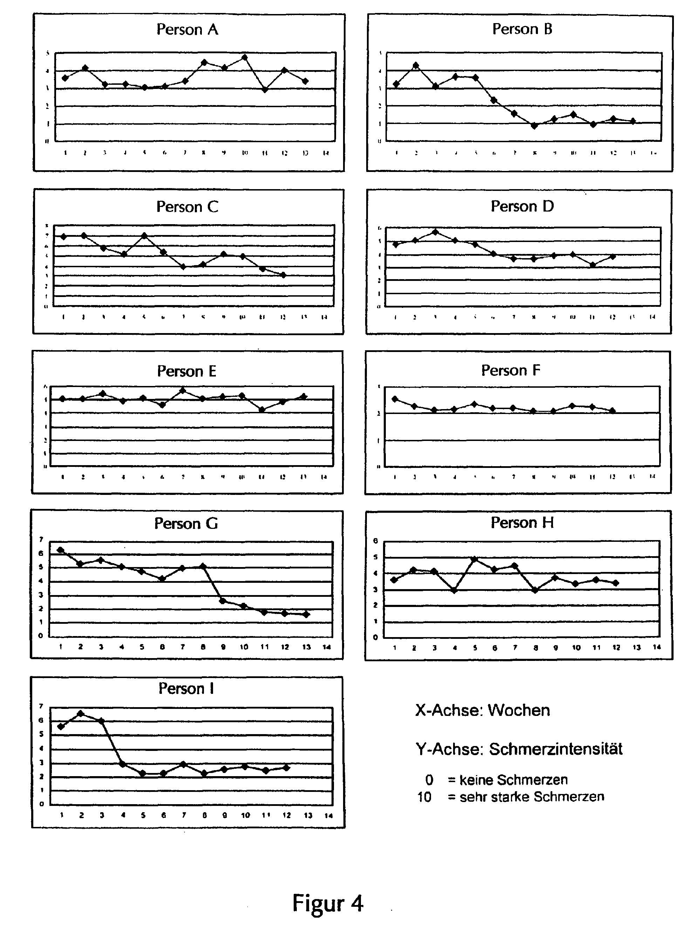

- **Fig. 4:**: zeigt die Einzelfallergebnisse in der Studie zur Magnetfeldtherapie im Bereich von Rückenerkrankungen über einen Verlauf von 12 Wochen in grafischer Form für Pa- tienten A bis I.
- **Fig. 5:**: zeigt Magnetfeldspulen. Hier wird ein üblicherweise industriell eingesetztes sehr flaches und leichtes Flachkabel als Flächenspule (der elektrischen Leiterbahn gemäß Hauptanspruch) für die erfindungsgemäße Sitz- oder Liegevorrichtung eingesetzt. Dabei wird ein Flachkabel oder ein mehrpoliger Leiter so verbunden, dass eine Spule zur Applikation der Magnetfeldtherapie entsteht, wobei ein im Anschaffungspreis günstiges Industriekabel verwendet werden kann und die so geformte Flächenspule zusätzlich extrem flach und extrem leicht wird.

### Beispiele:

Die folgenden Beispiele sollen die oben beschriebenen Gegenstände und die dadurch erhaltenen Effekte illustrieren und sind in keiner Weise einschränkend für den Gegenstand der Schutzansprüche.

### 1. Studie zur Magnetfeldtherapie im Bereich von Rückenerkrankungen Einfettung

Die im Folgenden beschriebene Studie zur Magnetfeldtherapie im Bereich von Rückenerkrankungen wurde an der Universität Karlsruhe am Institut für Sportwissenschaften über den Zeitraum von 12 Wochen durchgeführt.

Die Studie untersuchte die schmerzreduzierende Wirkung der Magnetfeld-Therapie im Bereich von Rücken- und Gelenkbeschwerden. Hierzu unterzogen sich 9 Personen (die Zahl war durch die Verfügbarkeit der Magnetfeld-Matten des Anmelders vorgegeben) in 12 Wochen täglich 2x8 Minuten einer induktive Behandlung.

Neben diversen Angaben zur Person wurden regelmäßig an vier Tageszeiten Schmerzzustände (morgens, mittags, abends, nachts) erfasst. Die Werte wurden gemittelt und as Wochenwerte in die Ergebnisdarstellung übernommen.

Wegen der geringen Zahl der Versuchspersonen lag der Schwerpunkt der Auswertung auf Einzelfallbeschreibungen. Hier interessierte vor allem die Entwicklung des Gesamtkurvenverlaufs. Dieser war tendenziell als Anstiegs- oder Abfallswinkel zu erkennen.

Zusätzlich wurde die gesundheitliche Eingangssituation mit der Ausgangssituation in Form einer Differenzbildung zwischen dem ersten und dem letzten Schmerzrating bzw. des gemittelten Wertes der ersten und letzten drei Schmerzratings verglichen.

### Ergebnisse

a) Vergleich der ersten und letzten Woche aller Teilnehmer
Vergleich der ersten und der letzten Woche aller Teilnehmer:

| Name | Wert erste Woche | Wert letzte Woche | Differenz |
|---|---|---|---|
| | | | |
| Person A | 6,61 | 3,39 | +0,22 |
| Person B | 3,25 | 1,11 | +2,14 |
| Person C | 6,89 | 3,10 | +3,79 |
| Person D | 4,72 | 3,80 | +0,92 |
| Person E | 5,10 | 3,00 | +2,10 |
| Person F | 2,54 | 2,09 | +0,45 |
| Person G | 6,30 | 1,64 | +4,66 |
| Person H | 3,61 | 3,00 | +0,61 |
| Person I | 5,64 | 2,32 | +3,32 |

| | | | |
|---|---|---|---|
| * Die positiven Vorzeichen sind als Schmerzreduktion zu interpretieren. | | | |

Vergleich der Mittelwerte der ersten drei und letzten drei Wochen aller Teilnehmer:

| Name | Wert erste drei Wochen | Wert letzte drei Wochen | Differenz |
|---|---|---|---|
| | | | |
| Person A | 3,92 | 3,66 | +0,26 |
| Person B | 4,23 | 1,24 | +2,99 |
| Person C | 6,56 | 3,95 | +2,61 |
| Person D | 5,17 | 3,66 | +1,51 |
| Person E | 5,22 | 4,83 | +0,39 |
| Person F | 2,31 | 2,20 | +0,11 |
| Person G | 5,73 | 1,94 | +3,79 |
| Person H | 3,98 | 3,45 | +0,53 |
| Person I | 6,07 | 2,67 | +3,40 |

| | | | |
|---|---|---|---|
| * Die positiven Vorzeichen sind als Schmerzreduktion zu interpretieren. | | | |

b) Gesamtergebnis

| Proband | Schmerzbereiche | Schmerzen seit | Bisherige Behandlung | Therapieerfolg |
|---|---|---|---|---|
| | | | | |
| Person A | kompletter Rücken | über 1 Jahr | 2 Besuche bei Facharzt; 1 Besuch bei Allgemeinmediziner | gleich geblieben |
| Person B | oberer (HWS) und unterer (LWS) Rücken | über 1 Jahr | 2 Besuche bei Facharzt; 2 Besuche bei Physiotherpeuten | stark verbessert |
| Person C | oberer (HWS) und unterer (LWS) Rücken | über 1 Jahr | Mehrere Besuche bei Facharzt und Allgemeinmediziner, Physiotherapeuten und Masseur | stark verbessert |
| Person D | oberer (HWS) und mittlerer (BWS) Rücken | über 1 Jahr | 5 Besuche bei Facharzt; 8 Besuche bei Allgemeinmediziner sowie 3 Besuche bei Physiotherpeuten | schwach verbessert |
| Person E | mittlerer (BWS) und unterer (LWS) Rücken | über 1 Jahr | Jeweils 10 Besuche bei Facharzt und Allgemeinmediziner, jeweils 3 Besuche bei Physiotherapeuten und Masseur | gleich geblieben |
| Person F | unterer (LWS) Rücken | über 6-12 Monate | 1 Besuch bei Facharzt | schwach verbessert |
| Person G | mittlerer (BWS) und unterer (LWS) Rücken | über 1 Jahr | Jeweils 5 Besuche bei Facharzt und Allgemeinmediziner, jeweils 2 Besuche bei Physiotherapeuten und Masseur | stark verbessert |
| Person H | oberer (HWS) Rücken | über 1 Jahr | Jeweils 4 Besuche bei Facharzt und Masseur | schwach verbessert |
| Person I | kompletter Rücken | 6-12 Monate | 15 Besuche bei Facharzt | stark verbessert |

### Zusammenfassung

Alle neun Personen klagten über Rückenschmerzen, bei drei Personen kamen Schmerzen im Knie- und Sprunggelenk sowie an der Halswirbelsäule hinzu. Bei allen Teilnehmern wurden körperliche und/oder psychische bzw. berufliche Belastungen als verursachende Faktoren für die Schmerzentwicklung angeführt. Ebenso waren alle Personen - bis auf eine Ausnahme - wiederholt beim Arzt, bzw. hatten zahlreiche Behandlungen (z.B. physiotherapeutischer Art) hinter sich. Die individuellen Kurvenverläufe betreffend kann festgestellt werden, dass bei:
- 2 Personen kein
- 3 Personen ein schwacher
- 4 Personen ein deutlicher
magnetfeldtherapeutischer Erfolg aufgetreten ist. Es handelt sich jeweils um individuelle Verlaufskurven, deren Profile teilweise auch durch spezifische Ereignisse zu erklären sind. So beispielsweise durch einen Sturz vom Pferd (Person H) der durch eine krankheits- oder urlaubsbedingte Behandlungsunterbrechung (Person G). Die Vergleichswerte der Eingangs- und Beendigungstermine ergänzen die Einzelfallauswertung. Demnach liegen alle Differenzwerte im positiven Bereich (Schmerzreduktion). Auch hier zeigen sich
a) im Vergleich der jeweils ersten und letzten Werte:
   - 5 deutliche Schmerzreduktionswerte
   - 2 schwache Schmerzreduktionswerte
b) im Vergleich der jeweils gemittelten ersten und letzten drei Werte:
   - 4 deutliche Schmerzreduktionswerte
   - 1 mittlerer Schmerzreduktionswert
   - 3 schwache Schmerzreduktionswerte

Eine zusammenfassende Bewertung bestätigt also den Erfolg der magnetfeldtherapeutischen Anwendung unter Verwendung einer der Erfindung entsprechenden aktiven Brückenschaltung im Bereich von Rückenbeschwerden in seiner Grundtendenz und besonders deutlich in 4 von 9 Einzelfällen.

Die Einzelfallergebnisse sind in grafischer Form für die Personen A bis I in Figur 4 gezeigt.

### 2. Überprüfung der Effekte einer Stimulation mit niedrigenergetischen elektromagnetischen Feldern auf die Proliferation von fünf verschiedenen Zellinien in vivo

Diese Studie wurde von der Internationalen Ärztegesellschaft für Energiemedizin, IGEM in Österreich und der Santerra Handels GmbH unterstützt und von der JSW-Research Forschungslabor GmbH und Institut für Embryologie und Histologie, Karl-Franzens-Universität Graz durchgeführt.

Im Rahmen dieser Studie sollten die Effekte einer niedrigenergetischen elektromagnetischen Stimulation (EMS; 40 µT, Sägezahnimpuls, Frequenzband) auf die Proliferation (Zellteilung) von isolierten Zellen 5 verschiedener Zeillinien untersucht werden.

Diese Studie wurde initiiert, da bereits in einer vorangegangenen orientierenden Pilotuntersuchung für IGM-Effekte einer solchen Stimulation auf das "Verhalten" von Nervenzellen nachgewiesen werden konnten. Nervenzellen, die sich ja nicht mehr teilen können, zeigten nachweislich einen leicht erhöhten Stoffwechsel aufgrund des EMS. Was aber für Nervenzellen, also für das Gehirn und periphäre Nervenbahnen, durchaus positiv ist, kann im Falle von Zellwucherungen, wie z.B. von Tumoren unerwünscht sein. Daher sollten auch bei JSW-Research unter Laborbedingungen die Fragestellung untersucht werden, inwieweit eine elektromagnetische Stimulation die Teilung von Tumorzellen beeinflusst.

Folgende Zellen wurden verwendet: die humane Adonokarzinomazellinie HeLa, die humane Choriokarzinomazellinien Jar und Jar-3, Neuro-2a, eine Maus-Neuroblastom-Zelllinie, sowie die humane Neuroblastom-Zelllinie SH-SY5Y. Ganz generell wurde in GLP (good laboratory practice) zertifizierten Labors, mit speziell geschultem Personal und unter standardisierten Bedingungen gearbeitet. Für jede Zellinie wurden spezielle Medien mit definierten Zuätzen hergestellt (HeLa, Jeg-3, und Neuro-2a: Eagle's minimum essential medium - EMEM - mit 2 mM L-Glutamin, 10% FCS und 1 % Gentamycin; Jar: RPMI 1640 mit 2 mM L-Glutamin, 10% FCS 1% Gentamycin; SH-SY5Y: EMEM und HAM's F12-Medium (1:1 Mischungsverhältnis) mit 10% FCS und 1 % Penstrep.). Die Zellen, jeweils 10.000 pro ml Nährmedium, jeder der oben genannten Zelllinien wurden in für die adäquate Medien in 96-Well-Zellkulturplatten ausgesät und dann in einem CO₂-Inkubator unter für isolierte Zellen optimalen *in vitro* Bedingungen (37°C, 95% Luftfeuchte und 5% CO₂) gehalten. Entweder wurden Zellen in einem Inkubator mit oder zu Kontrollzwecken ohne eine erfindungsgemäße Vorrichtung in Kissenform im Gerät, d.h. ohne Stimulation kultiviert. Die halbmaximale EM-Stimulierung (40 µT) dauerte jeweils 24 Minuten. Gestartet wurde damit am Tag 1 nach der Präparation der Zellkulturplatten um 15.00 Uhr, danach wurde jeweils um 7.00 Uhr und um 15.00 Uhr stimuliert (d.h.: Tag 1: 15.00 Uhr, Tag 2: 7.00 Uhr und 15.00 Uhr, Tag 3: 7.00 Uhr und 15.00 Uhr, Tag 4: 7.00 Uhr). Die Inkubationszeiten für die Zellkulturplatten dauerten jeweils für drei verschiedene Zeiten nämlich für 20, 44 und 68 Stunden (d.h. 20 Stunden - 2 x EMS, 44 Stunden - 4 x EMS und 68 Stunden - 6 x EMS) und am Ende dieser Zeiten wurde einerseits die Zellproliferation oder die Zellteilung (MTT-Test) andererseits auch das Maß für die Anzahl möglicherweise unter elektromagnetische Stimulation abgestorbener Zellen (LDH-Test) gemessen. Sowohl der MTT-Test als auch der LDH-Test sind für Zellkulturexperimente gängige biochemische Farbreaktionen.

Basierend auf den Ergebnissen dieser Studie konnte festgestellt werden, dass eine schwache elektromagnetische Stimulation (Magnetfeldpolster, Intensität: halbmaximal für 24 Minuten [40 µT, Sägezahnimpuls, Frequenzband] und maximal 2 mal täglich) keinen Einfluss auf die Proliferation der hier ausgetesteten Tumorzelllinien HeLa (humanes Adenokarzinom), Jar und Jeg-3 (humanes Choriokarzinom), Neuro-2a (Maus-Neuroblastom) sowie SH-SY5Y (humanes Neuroblastom) hat.

### 3. Studie der Effekte einer Stimulation mit niedrigenergetischen elektromagnetischen Feldern auf verschiedene Einzelwerte am Menschen

In einer weiteren Studie wurde die erfindungsgemäße Vorrichtung an Probanden in einem Belastungstest untersucht, um den Einfluss der Magnetfeldtherapie auf Faktoren wie Kraftverfügbarkeit, Reaktionszeit, Gleichgewicht, Herzzustand, Stressbelastung, Pulsrate, etc. zu bestimmen.

Die Ergebnisse sind im Folgenden tabellarisch zusammengefasst:

| | Männer | | Verbesserung mit erfindungsgemäßer. Technologie | Frauen | | Verbesserung mit erfindungsgemäßer. Technologie |
|---|---|---|---|---|---|---|
| Zusammenfassung | Kontrollgruppe | Gruppe mit Magnetfeldtherapie | | Kontrollgruppe | Gruppe mit Magnefeldtherapie | |
| | nach 2. Messung | nach 2. Messung | | nach 2. Messung | nach 2. Messung | |
| Kraftverfügbarkeit, re. | -1 | 1,10 | 2 | -4 | 4 | 8 |
| Kraftverfügbarkeit, li. | -5 | 1,10 | 6 | -2 | 3 | 5 |
| Reaktionszeit, verkürzt | 1 | 4 | 3 | 1 | 6 | 5 |
| Länge Weg Schwerpunktgewicht | -14 | 4 | 18 | -17 | 7 | 24 |
| Herzzustand | -2 | 7 | 9 | 1 | 1 | 0 |
| Stressbelastung | -21 | 6 | 27 | -19 | 17 | 36 |
| Pulsrate | -1 | 2 | 3 | 5 | 6 | 1 |

| | | | | | | |
|---|---|---|---|---|---|---|
| (Alle Angaben in %) | | | | | | |

## Patentansprüche

1. Sitz- oder Liegevorrichtung zur Erzeugung eines Magnetfeldes zur Magnetfeldtherapie, umfassend
a) mindestens einen Aufnahmebereich;
b) eine Einrichtung, umfassend eine elektrische Leiterbahn, die zur bipolaren Stromerregung zur Erzeugung periodischer modulierter Magnetfelder im Aufnahmebereich eingerichtet ist;
c) eine aktive Brückenschaltung zur Steuerung der bipolaren Stromerregung zur Erzeugung periodischer modulierter Magnetfelder;
d) eine programmierbare Steuerung, die die Steuerung der Erzeugung periodischer modulierter Magnetfelder ermöglicht;
e) optional einen Sensor, der bevorzugt mit der Einrichtung nach b), der aktiven Brückenschaltung nach c) und/oder der Steuerung nach d) verbunden ist;
f) optional eine Energiequelle, die bevorzugt mit der der Einrichtung nach b), der aktiven Brückenschaltung nach c), der Steuerung nach d) und/oder dem Sensor nach e) verbunden ist.

2. Sitz- oder Liegevorrichtung zur Erzeugung eines Magnetfeldes zur Magnetfeldtherapie, umfassend
a) mindestens einen Aufnahmebereich;
b) eine Einrichtung, umfassend eine elektrische Leiterbahn, die zur bipolaren Stromerregung zur Erzeugung periodischer modulierter Magnetfelder im Aufnahmebereich eingerichtet ist;
c) optional eine aktive Brückenschaltung zur Steuerung der bipolaren Stromerregung zur Erzeugung periodischer modulierter Magnetfelder;
d) eine programmierbare Steuerung, die die Steuerung der Erzeugung periodischer modulierter Magnetfelder ermöglicht;
e) einen Sensor, der bevorzugt mit der Einrichtung nach b), der aktiven Brückenschaltung nach c) und/oder der Steuerung nach d) verbunden ist;
f) optional eine Energiequelle, die bevorzugt mit der der Einrichtung nach b), der aktiven Brückenschaltung nach c), der Steuerung nach d) und/oder dem Sensor nach e) verbunden ist.

3. Sitz- oder Liegevorrichtung nach Anspruch 1 oder 2, wobei die Sitz- oder Liegevorrichtung eine Sitzvorrichtung ist, ausgewählt aus einem Autositz, insbesondere einem Autositz für Personenkraftwagen, Lastkraftwagen, Busse, und Nutzfahrzeuge, einem Flugzeugsitz, Flugbegleiter- bzw. Cockpitsitze, einem Sitzmöbel aus dem Wohnbereich oder Arbeitsbereich, einem Stuhl, einem Fernsehsessel, einem Couchsessel, einem Bürostuhl, und/oder einem ärztlichen Behandlungsstuhl.

4. Sitz- oder Liegevorrichtung nach einem der Ansprüche 1 bis 3, wobei die Liegevorrichtung ausgewählt ist aus einer Liege oder Matratze, umfassend eine Liege oder Matratze für den häuslichen Gebrauch, eine Liege oder Matratze für den ärztlichen oder therapeutischen Gebrauch, eine Krankenhausliege oder - matratze, und/oder eine Massageliege oder -matratze.

5. Sitz- oder Liegevorrichtung nach einem der Ansprüche 1 bis 4, wobei der Aufnahmebereich in der Sitzvorrichtung in einer Fußstütze, dem Beinbereich, der Sitzfläche, der Rückenlehne und/oder der Kopfstütze der Sitzvorrichtung enthalten ist, oder in der Liegevorrichtung in der Fußfläche, dem Beinbereich, dem Hüftbereich, dem Rückenbereich und/oder dem Kopfbereich der Liege oder Matratze enthalten ist.

6. Sitz- oder Liegevorrichtung nach einem der Ansprüche 1 bis 5, wobei die elektrische Leiterbahn ein mehradriges Flachkabel, eine Superlight-Spule, eine Superlight-Spule gefertigt aus mehradrigen Flachkabeln, ein Flexleiter, ein Flexleiter gefertigt aus mehradrigen Flachkabeln, oder eine daraus gefertigte Spule, oder eine sonstige geeignete Applikatorspule umfasst, die jeweils geeignet ist, durch bipolare Stromerregung ein periodisches moduliertes Magnetfelder zu erzeugen.

7. Sitz- oder Liegevorrichtung nach einem der Ansprüche 1 bis 6, wobei die aktive Brückenschaltung zur Steuerung der bipolaren Stromerregung zur Erzeugung periodischer modulierter Magnetfelder **dadurch gekennzeichnet ist, dass** zwei P-Kanal-MOSFET (1, 3), zwei N-Kanal-MOSFET (2, 4) und zwei Widerstände (15, 20) in einer Brücke (B) integriert sind, die Quellen (S1, S3) von P-Kanal-MOSFET (1, 3) zu der positiven Speisespannung geschaltet sind, der Drain (D1) von P-Kanal-MOSFET (1) und der Drain (D2) von N-Kanal-MOSFET (2) gekoppelt sind, der Drain (D3) von P-Kanal-MOSFET (3) und der Drain (D4) von N-Kanal-MOSFET (4) zueinander geschaltet sind, zu den Ausgängen der Brückenschaltung (22, 23) eine Applikatorspule (21) gekoppelt ist, zwei Steuereinheiten (I, III) zu den P-Kanal-MOSFET (1, 3) und zwei Steuereinheiten (II, IV) zu den N-Kanal-MOSFET (2, 4) geschaltet sind, die Steuereinheiten (III, IV) durch das Kabel des Enable-Signals "A" (24) und die Steuereinheiten (I, II) durch das Kabel des Enable-Signals "B" (25) verbunden sind, das für die Eingangssignale vorgesehene Kabel (26) zu den Steuereinheiten (II, IV) gekoppelt ist und die Steuereinheiten (II, IV), sowie die Widerstände (15, 20) mit einem Erdungskontakt (GND) der Brücke (B) verbunden sind.

8. Sitz- oder Liegevorrichtung nach Anspruch 7, wobei die aktive Brückenschaltung zur Steuerung der bipolaren Stromerregung zur Erzeugung periodischer modulierter Magnetfelder **dadurch gekennzeichnet ist, dass** der Eingangspunkt EN-B durch Widerstände (12, 13) zu Transistoren (5, 6) geschaltet ist, der Kollektor (K5) des Transistors (5) einerseits durch einen Widerstand (11) zur positiven Speisespannung (+), andererseits zu dem Tor (G1) des P-Kanal-MOSFET (1) gekoppelt ist, die Quellen (S1, S3) der P-Kanal-MOSFET (1, 3) zur positiven Speisespannung (+) geschaltet sind, der Eingangspunkt EN-A durch Widerstände (17, 18) zu Transistoren (7, 8) geschaltet ist, der Kollektor (K7) des Transistors (7) einerseits MOSFET (3) gekoppelt ist, der Eingang (A-input) durch Widerstände (14, 19) einerseits zu Kollektoren (K6, K8) von Transistoren (6, 8), andererseits zu nicht invertierenden Eingängen (03, 05) von Operationsverstärkern (9, 10) geschaltet ist, die Ausgangspunkte (O1 07) von Operationsverstärkern (9, 10) zu Toren (G2, G4) der N-Kanal-MOSFET (2,4) gekoppelt sind, die invertierenden Eingänge (02, 06) zu den Quellen (S2, S4) der N-Kanal- MOSFET (2, 4), sowie zu Widerständen (15, 20) geschaltet sind, die Emitter (E5, E6, E7, E8) der Transistoren (5, 6, 7, 8) und die Widerstände (15, 20) mit dem Erdungskontakt (GND) verbunden sind.

9. Sitz- oder Liegevorrichtung nach einem der Ansprüche 1 bis 8, wobei die programmierbare Steuerung zur Erzeugung periodischer modulierter Magnetfelder eine individuell programmierbare und/oder eine vorprogrammierte Einstellung mit Auswahl von verschiedenen Parametern ermöglicht.

10. Sitz- oder Liegevorrichtung nach Anspruch 9, wobei die individuell programmierbare und/oder eine vorprogrammierte Einstellung die Einstellung der Magnetfeldstärke, die Dauer der Behandlung, der Körpergröße, des Körpergewichts, der Dynamik der Magnetfeldstärke über die Dauer der Behandlung, Einstellungen zu der zu behandelnden Körperzone, die Tageszeit und davon abhängige Programmierungen und Einstellungen umfasst.

11. Sitz- oder Liegevorrichtung nach Anspruch 9 oder 10, wobei die programmierbare Steuerung zur Erzeugung periodischer modulierter Magnetfelder intern an oder in der Sitz- oder Liegevorrichtung befestigt ist oder extern, und bevorzugt ein Panel, ein Bedienfeld, eine Datenspeichervorrichtung und/oder eine (Benutzer-)Schnittstelle umfasst.

12. Sitz- oder Liegevorrichtung nach einem der Ansprüche 1 bis 11, wobei (pulsierende) elektromagnetische Feldstärken mit maximalen Flussdichten der Applikator-Abstrahlungen von 1 bis 10.000 µT, bevorzugt 10-800 µT, noch stärker bevorzugt 10-600 µT und am stärksten bevorzugt zwischen 10 und 100 µT erzeugt werden.

13. Sitz- oder Liegevorrichtung nach einem der Ansprüche 1 bis 12, wobei zur Entspannung eine (pulsierende) elektromagnetische Feldstärke mit maximalen Flussdichten der Applikator-Abstrahlungen von etwa 10 bis 40 µT verwendet werden, zur Aktivierung eine (pulsierende) elektromagnetische Feldstärke mit maximalen Flussdichten der Applikator-Abstrahlungen von etwa 30 bis 100 µT verwendet werden, zur Behandlung von Schmerz eine (pulsierende) elektromagnetische Feldstärke mit maximalen Flussdichten der Applikator-Abstrahlungen von etwa 10 bis 800 µT verwendet werden, und zur Behandlung von Gelenken eine (pulsierende) elektromagnetische Feldstarke mit maximalen Flussdichten der Applikator-Abstrahlungen von etwa 10 bis 10.000 µT verwendet werden.

14. Sitz- oder Liegevorrichtung nach einem der Anspruche 1 bis 13, wobei die pulsierende elektromagnetische Feldstarke einen sinusförmigen -, einen Rechteck-, einen Sägezahn -, einen inversen Sagezahnimpuls oder Frequenzband, aufweist oder eine Hüllkurve aus diesen Signalen, Impulsen oder Frequenzbändern; und/oder
wobei die pulsierende elektromagnetische Feldstarke in einem Frequenzbereich zwischen etwa 1 und 300 Hz auftritt; und/oder
wobei der Sensor die bipolare Stromerregung zur Erzeugung periodischer modulierter Magnetfelder bei Benutzung anschaltet und bei Nichtbenutzung wieder ausschaltet; und/oder
wobei der Sensor ein Erkennungssensor ist, ausgewählt aus einem Drucksensor, einem Infrarotsensor, einem Ultraschallsensor, einem optischen Sensor, einem optischen Sensor, der mit einer Lichtschranke verbunden ist, einen elektrischen Kontakt- oder Nahesensor, oder einem Sensor, der auf Veränderung des Dipolmomentes oder des Widerstandes reagiert; und/oder
wobei die Sitz- oder Liegevorrichtung zusätzlich einen Biofeedbacksensor aufweist, die zum Erfassen von einem physiologischen Parameter eines Nutzers ausgestaltet ist, einschließlich eines Finger-Sensors; und/oder
wobei die Sitz- oder Liegevorrichtung eine Anzeigevorrichtung aufweist; und/oder
wobei die Energiequelle extern oder intern vorliegt.

15. Sitz- oder Liegevorrichtung nach einem der Anspruche 1 bis 20, wobei die Sitz oder Liegevorrichtung in einem Rollstuhl, für Matratzen (Matratzenapplikation), für Privatanwender, bei Kassen- und Privatpatienten in der Klinik, in Alters,- und Pflegeheimen, bei Kur- und Wellnesseinrichtungen, im Bereich Luftfahrt (Aircraft), insbesondere dort im geschäftlichen Bereich, bei Schiffsreisen, bei Thrombosepatienten, zur Jetlag-Prophylaxe, zum Lösen von Verspannungen durch langes Sitzen, im Eisenbahnbereich, insbesondere dort im geschäftlichen Bereich, zum Lösen von Verspannungen durch das lange Sitzen, bei Automotive-Anwendungen, zur Steigerung der Konzentration und Aufmerksamkeit, zum Lösen von Verspannungen bei längeren Autofahrten, im Bürobereich (Office), zur Regeneration und zum Stressabbau, zur Erhöhung der Leistungsbereitschaft, zur Erhöhung des allgemeinen Wohlgefühls und somit der Motivation, und zur Erhöhung der Konzentrationsfähigkeit eingesetzt wird.
